Europäisches Patentamt

European Patent Office

Office européen des brevets

(19)

(11) **EP 1 536 236 A1**

(12) **EUROPEAN PATENT APPLICATION**

(43) Date of publication:
**01.06.2005 Bulletin 2005/22**

(51) Int Cl.⁷: **G01N 33/86**

(21) Application number: **04028118.0**

(22) Date of filing: **26.11.2004**

| | |
|---|---|
| (84) Designated Contracting States:<br>**AT BE BG CH CY CZ DE DK EE ES FI FR GB GR<br>HU IE IS IT LI LU MC NL PL PT RO SE SI SK TR**<br>Designated Extension States:<br>**AL HR LT LV MK YU** | (72) Inventors:<br>• **Okuda, Masahiro**<br>  **Kobe-shi Hyogo 651-2214 (JP)**<br>• **Hoshiko, Susumu**<br>  **Kobe-shi Hyogo 651-2217 (JP)** |
| (30) Priority: **28.11.2003 JP 2003398563** | (74) Representative: **HOFFMANN - EITLE**<br>**Patent- und Rechtsanwälte**<br>**Arabellastrasse 4**<br>**81925 München (DE)** |
| (71) Applicant: **Sysmex Corporation**<br>**Kobe-shi, Hyogo 651-0073 (JP)** | |

(54) **Reagent for measuring clotting time and method for measuring clotting time**

(57) The present invention provides a reagent for measuring clotting time that allows accurate analysis of blood coagulation in samples containing antiphospholipid antibodies, wherein the clotting time is measured by using a sample containing an antiphospholipid antibody, a causative substance of antiphospholipid antibody syndromes, and the reagent for measuring clotting time containing antibody, serum, plasma and immunoglobulin of vertebrates except human.

EP 1 536 236 A1

**Description**

FIELD OF THE INVENTION

The present invention relates to a reagent for measuring blood coagulation for use as a clinical laboratory test and a method for measuring clotting time.

BACKGROUND

Measurements of prothrombin time (hereinafter, simply referred to as "PT") and combined factors are routinely practiced as screening tests in the field of blood coagulation diagnosis. In measurements of PT, plasmas from patients are mixed with a reagent at least containing a tissue factor and calcium ion. In addition, in measurements of the combined factors, plasmas from patients are mixed with a reagent at least containing a tissue factor, factor V, fibrinogen and calcium ion. The tissue factors can be purified from tissues such as brain, lung and the like, or produced by genetic engineering technology (J.H. Lawson et. al., Method Enzymol. 222:177-180 (1993)). These tissue factors activate extrinsic blood coagulation pathway.

The PT and the combined factors are used for screening of the extrinsic blood coagulation system and for analysis of oral anticoagulants such as warfarin and the like. The PT measurement allows comprehensive analysis of the activities of the coagulation factors involved in the extrinsic coagulation system, factors II, V, VII, and X, and thus are widely used as a screening test before the coagulation tests and as a test for determining the effects of oral anticoagulants. The PT measurements are carried out by adding a test blood plasma into an analytical reagent containing a tissue factor and an adequate concentration of calcium ion as the main ingredients and determining the clotting time therein. On the other hand, the combined factor measurements are carried out by adding a test plasma into an analytical reagent containing a tissue factor, factor V, fibrinogen and an adequate concentration of calcium ion as the main ingredients and determining the clotting time therein.

A tissue factor-containing reagent characterized by containing a nickel compound for improvement in the sensitivity in determining the activities of coagulation factors with the reagents containing a tissue factor, a method for determining prothrombin time by using the composition, and a prothrombin time measurement reagent were already known (Japanese Patent Application Laid-open No. 2001-255332).

Antiphospholipid antibody is a general term indicating an antibody that binds to a complex between a lipid having negative charges such as phosphatidylserine and the like and β2-GPI or prothrombin, and a typical example thereof is a lupus anticoagulant (hereinafter, simply referred to as "LA"). Diseases positive in either of them and exhibiting one of a variety of characteristic clinical symptoms such thrombosis, abortion/premature delivery, or thrombocytopenia are called antiphospholipid syndromes (APSs). Recently it was found that an LA, one of the causative substances for the antiphospholipid antibody syndromes, is a heterogeneous antibody and a composite antibody binding to the complex between a negative charge phospholipid (phosphatidylserine) and β2-GPI or prothrombin. LA is also known as an immunoglobulin that inhibits phospholipid-dependent coagulation reactions without suppressing the activities of individual coagulation factors (Antiphospholipid Syndrome, 1st Ed., P.40, Juzo Matsuda, published by Shinkoh Igaku Shuppan Co., Ltd.).

Considering the facts described above, if an examinee is an antiphospholipid antibody carrier, results of PT and combined factor measurements in clinical laboratory tests are undeniably under the influence of the antiphospholipid antibodies. In particular, in recent progress in recombinant technology, more and more recombinant tissue factors have been used as the tissue factor for use in PT and combined factor measurements. The tissue factor prepared by the recombinant technology are characteristically higher in purity than conventional serum-derived tissue factors. In conventional reagents, the influence on the PT and combined factor measurements seem to be reduced by the reaction between an large excess of phospholipid and antiphospholipid antibodies such as LA and the like in test samples, but use of the tissue factors higher in purity enhanced the influence of the antiphospholipid antibodies contained in samples on PT and combined factor measurements. In particular, when warfarin is monitored in LA-positive or APS patients, it was difficult to obtain accurate coagulation activity because the phospholipid in reagents is adsorbed on LA in samples and thus there was a need to overcome such problems (Tripodi A. et al., Br. J. Haematol. 2001:115 (3); 672-8).

Therefore, there exists a demand for a method of measuring PT or combined factors accurately without the need for prior tests for determining whether the examinee has antiphospholipid antibodies.

SUMMARY

An object of the present invention is to provide a clotting time-measuring reagent that allow an accurate measurement with a sample containing antiphospholipid antibodies

A first aspect of the present invention relates to a reagent for measuring clotting time, comprising calcium ion, a tissue factor and a non-human-derived component selected from the group consisting of antibody, serum, plasma, and immunoglobulin which derive from vertebrates except human.

A second aspect of the present invention relates to a method for measuring clotting time, comprising the steps of

mixing a sample with a reagent comprising calcium ion, a tissue factor and a non-human-derived component selected from the group consisting of antibody, serum, plasma and immunoglobulin which derive from vertebrates except human,

and measuring clotting time of the mixture.

DESCRIPTION OF THE PREFRRED EMBODIMENT

In an embodiment, the reagent for measuring clotting time contains calcium ion, a tissue factor, and at least one ingredient selected from the group consisting of antibody, serum, plasma and immunoglobulin from vertebrates excluding human.

As described above, PT or combined factor measurement is the most commonly practiced screening test in the field of coagulation diagnosis. The combined factor is measured combined factor-measuring reagent. The combined factor measurement allows comprehensive analysis of factors II, VII and X. The test has been performed for monitoring oral anticoagulant as described above, but it was necessary to test separately whether the examinee of the test is an antiphospholipid antibody carrier. If the examinee is found to be an antiphospholipid antibody carrier in the test, it is possible to take actions to minimize the influence by the antiphospholipid antibody in the PT or combined factor measurement, but commonly it is not certain whether the examinee is previously tested before the PT or combined factor measurement. Therefore, if a measurement system that can suppress only the influence by antiphospholipid antibodies and exerts no other influences during the PT or combined factor measurement is available, it becomes possible to do the analysis without the need for the test for determining whether the patient is with antiphospholipid antibody syndromes.

Specific examples of the antibodies, sera, plasmas and immunoglobulins (hereinafter, collectively referred to as "non-human-derived component" when they are not differentiated) which derive from vertebrates except human include mouse IgG, equine IgG, bovine IgG, mouse serum, equine serum, bovine serum, mouse, equine or bovine $\alpha$-globulin, HBR (Human anti-Mouse Antibodies Blocking Reagent: manufactured by Scantibodies), NMS, MAK33 (manufactured by Roche), and the like. Favorable examples thereof include antibodies, sera, immunoglobulins and the like derived from mammal animals excluding human and pig. It becomes possible to reduce the influence by antiphospholipid antibodies on clotting time measurement by allowing an adsorption or neutralization reaction to progress between the non-human-derived component and the antiphospholipid antibodies

The non-human-derived component may be mixed previously into the prothrombin time (PT)-measuring or combined factor-measuring reagent. The amount of the non-human-derived component added is 5 to 100 $\mu$g and preferably 10 to 50 $\mu$g per test (sample amount: 10 to 20 $\mu$l). The non-human-derived component may be used alone or in combination of two. Specifically, the non-human-derived component may be added at an amount of 100 to 500 $\mu$g per 10 ml of the PT-measuring reagent. On the other hand, the non-human-derived component may be added at an amount of 5 to 200 $\mu$g per 10 ml of the combined factor-measuring reagent. Alternatively, the non-human-derived component may be added to the samples for measurement of clotting time before use. In such a case, a common PT- or combined factor-measuring reagent may be used. The non-human-derived component may also be used alone or in combination of two in this case.

The measurements may be performed under a condition that the human antiphospholipid antibody contained is selectively adsorbed on the non-human-derived component, by mixing the sample and the non-human-derived component. For example, the time required for the adsorption of human LA is 1 to 30 minutes, preferably 2 to 10 minutes, and more preferably approximately 5 minutes; the favorable pH, 6 to 8; the favorable temperature, 30 to 40°C; and the salt concentration, 1 to 500 mM.

Samples used in measurement are generally plasmas. In addition, blood-derived substances prepared for general blood coagulation measurement systems may also be used. In other words, plasma or serum components obtained by separating blood samples collected from examinees with or without use of an anticoagulant such as heparin or the like, according to any one of common separation methods such as centrifugation and the like may be used as the sample. The plasma and serum components may be further purified by ultracentrifugation, and a particular fraction thereof may be used as a sample, for suppression of the nonspecific adsorption dependent on the measuring method and thus for allowing high-accuracy measurement.

A PT-measuring reagent containing non-human-derived component, a tissue factor and calcium ion, a PT-measuring reagent kit having a first reagent containing non-human-derived component and a tissue factor and a second reagent containing calcium ion, and the like may be used as the PT-measuring reagent. In addition, a combined factor-measuring reagent containing non-human-derived component, a tissue factor, factor V, fibrinogen and calcium ion, a combined factor-measuring reagent kit having a first reagent containing non-human-derived component, a tissue factor, factor V and fibrinogen and a second reagent containing calcium ion; and the like may be used as the combined factor-measuring reagent. Examples of the tissue factors include those extracted or purified from biological samples such as brain, lung and the like and those prepared by recombinant technology. Relipidated tissue factor compositions, which reconstitute a tissue factor prepared by recombinant technology and lipidated with a phospholipid, may also be used. Further, the tissue factors may be derived, for example, from human, rabbit, calf, monkey, and the like. Purified substances may be used respectively as the factor V and fibrinogen, but plasmas treated by barium sulfate adsorption are usually used. Plasma treated by barium sulfate adsorption can be prepared by adding approximately 10 to 30 (W/V) % barium sulfate into a bovine blood plasma, mixing the mixture at room temperature for about 20 minutes, and removing the barium sulfate. The treated blood plasma is practically free from at least coagulation factors II, VII and X and retains desirable factor V and fibrinogen. The PT-measuring and combined factor-measuring reagents may be either a lyophilized reagent or a liquid reagent. Lyophilized reagents are used as dissolved in purified water or a buffer solution before use, and the non-human-derived component may be added to this purified water or buffer solution. In such a case, it is not necessary to add the non-human-derived component into the PT- or combined factor-measuring reagent in the lyophilized form.

In addition to the PT- and combined factor-measuring reagents described above, the present invention can also be applied to APTT (activated partial thromboplastin time)-measuring reagents.

EXAMPLE

Hereinafter, the present invention will be described with reference to Examples, but it should be understood that the invention is not limited thereto.

(Example 1)

(Preparation of reagents)

To a commercially available PT reagent (ThromboCheck PT, manufactured by Sysmex (reagent 1)) bottle, 1 mL of 2 mg/mL HBR (Human anti-Mouse Antibodies Blocking Reagent, manufactured by Scantibodies)(A) and then 9 mL of purified water were added to make a total volume of 10 mL. Separately, 1 mL of physiological saline and then 9 mL of purified water were added in a similar manner to the same bottle, to give an HBR-free solution (B). In a similar manner, HBR-containing reagents (A) and HBR-free reagents (B) of another commercially available recombinant PT reagent, Innovin (manufactured by Dade Behring, Germany (reagent 2)), and of a human placental tissue factor PT reagent, Thromborel S (manufactured by Dade Behring, Germany (reagent 3)), were prepared.

(Method for measurement)

100μL of the reagent (A) and reagent (B) prepared from each of the above-mentioned three kinds of PT reagents were added to a plasma sample (50 μL), and the mixture was analyzed twice by using a full automatic blood coagulation analyzer Coagrex 800 (Shimadzu Corporation). Coagutrol N (manufactured by Sysmex) was used as normal plasma, while LA-positive patient plasma (purchased from George King Bio-Medical, Inc. and ADI), warfarin-administered patient plasma (purchased from George King Bio-Medical, Inc.), LA positive warfarin-administered patient plasma (purchased from George King Bio-Medical, Inc.), heparin-administered patient plasma (Sunfco Ltd.), and factor II-deficient patient plasma (purchased from George King Bio-Medical, Inc.) were used as test samples.

In addition, INR is calculated according to the following formula:

$$INR = (PT \text{ of test sample} / PT \text{ of normal plasma})^{ISI}$$

(Results)

The results are summarized in Table 1.

In measuring the plasmas from the patients in the warfarin-administered LA-positive patient group, the clotting time was shortened more significantly in the HBR-containing system (A) than in the HBR-free system (B). On the other hand, in measurement of normal plasmas and the plasmas from warfarin-administered patients, heparin-administered patients, factorII-deficient patients, and no warfarin and no heparin-administered LA-positive patients, no change in clotting time or INR was observed both in the HBR-free reagent system (B) and the HBR-containing reagent system (A).

On the other hand, when the lupus anticoagulants (LA) in the plasmas of warfarin-administered LA-positive patients were analyzed by dRVVT method (dilute Russell's viper venom time, manufactured by Medical & Biological Laboratories Co., Ltd.), all plasmas were shown to be positive at a value higher than the cutoff value of 1.3.

These results indicate that the HBR-containing PT reagent neutralizes LA and thus allows accurate warfarin monitoring.

Table 1

| | Reagent 1 | | | | Reagent 2 | | | | Reagent 3 | | | | LA measurement (dRVVT) |
| | HBR-free (B) | | HBR-containing (A) | | HBR-free (B) | | HBR-containing (A) | | HBR-free (B) | | HBR-containing (A) | | |
| | (Sec) | (INR) | (Sec) | (INR) | (Sec) | (INR) | (Sec) | (INR) | (Sec) | (INR) | (Sec) | (INR) | |
|---|---|---|---|---|---|---|---|---|---|---|---|---|---|
| Normal plasma 1 | 11.9 | 1.00 | 12.0 | 1.00 | 12.6 | 1.00 | 12.7 | 1.00 | 10.5 | 1.00 | 10.6 | 1.00 | 1.03 |
| Normal plasma 2 | 10.2 | 0.84 | 10.4 | 0.85 | 11.5 | 0.88 | 11.4 | 0.86 | 10.1 | 0.96 | 10.2 | 0.96 | 1.09 |
| Normal plasma 3 | 11.4 | 0.95 | 11.6 | 0.96 | 12.3 | 0.97 | 12.6 | 0.99 | 11.2 | 1.07 | 10.7 | 1.01 | 0.98 |
| Warfarin patient plasma 1 | 34.1 | 3.29 | 34.6 | 3.31 | 28.6 | 3.07 | 30.1 | 3.26 | 33.8 | 3.22 | 34.6 | 3.26 | 1.13 |
| Warfarin patient plasma 2 | 26.5 | 2.47 | 27.1 | 2.51 | 24.6 | 2.50 | 24.7 | 2.49 | 25.3 | 2.41 | 26.3 | 2.48 | 1.27 |
| Heparin patient plasma 1 | 12.4 | 1.05 | 12.3 | 1.03 | 12.4 | 0.98 | 12.6 | 0.99 | 11.2 | 1.07 | 10.7 | 1.01 | 1.09 |
| Heparin patient plasma 2 | 13.7 | 1.17 | 13.9 | 1.18 | 14.3 | 1.19 | 14.2 | 1.17 | 12.6 | 1.20 | 12.2 | 1.15 | 1.16 |
| Factor II-deficient patient plasma 1 | 43.2 | 4.29 | 43.1 | 4.24 | 36.2 | 4.25 | 35.6 | 4.10 | 44.6 | 4.25 | 44.4 | 4.19 | 1.18 |
| Factor II-deficient patient plasma 2 | 33.8 | 3.25 | 34.2 | 3.27 | 49.8 | 6.57 | 49.5 | 6.45 | 85.8 | 8.17 | 86.9 | 8.20 | 1.09 |
| LA warfarin patient plasma 1 | 22.4 | 2.04 | 20.6 | 1.84 | 20.1 | 1.90 | 19.9 | 1.85 | 20.8 | 1.98 | 19.4 | 1.83 | 1.74 |
| LA warfarin patient plasma 2 | 18.4 | 1.64 | 16.7 | 1.45 | 17.7 | 1.59 | 16.5 | 1.43 | 17.4 | 1.66 | 15.3 | 1.44 | 1.35 |
| LA warfarin patient plasma 3 | 54.1 | 5.54 | 50.9 | 5.12 | 42.7 | 5.32 | 40.8 | 4.95 | 58.8 | 5.60 | 54.3 | 5.12 | 1.57 |
| LA warfarin patient plasma 4 | 44.2 | 4.41 | 42.1 | 4.13 | 37.6 | 4.47 | 33.9 | 3.84 | 48.6 | 4.63 | 44.2 | 4.17 | 1.76 |
| LA-positive patient plasma 1 | 13.2 | 1.12 | 13.7 | 1.16 | 15.6 | 1.34 | 15.7 | 1.34 | 12.5 | 1.19 | 12.3 | 1.16 | 2.19 |
| LA-positive patient plasma 2 | 33.1 | 3.18 | 33.7 | 3.21 | 30.2 | 3.31 | 30.2 | 3.28 | 36.9 | 3.51 | 34.5 | 3.25 | 1.53 |
| LA-positive patient plasma 3 | 12.1 | 1.02 | 12.4 | 1.04 | 14.3 | 1.19 | 14.1 | 1.15 | 10.7 | 1.02 | 10.8 | 1.02 | 1.86 |

## EP 1 536 236 A1

**Claims**

1. A reagent for measuring clotting time, comprising:

   calcium ion, a tissue factor and a non-human-derived component selected from the group consisting of antibody, serum, plasma and immunoglobulin which derive from vertebrates except human.

2. The reagent for measuring clotting time according to Claim 1, comprising first and second reagents, the first reagent including the tissue factor and the non-human-derived component, and the second reagent including the calcium ion.

3. The reagent for measuring clotting time according to any of Claim 1 or 2, wherein the reagent is a prothrombin time-measurement reagent.

4. The reagent for measuring clotting time according to Claim 1, wherein the concentration of the non-human-derived component is 10 to 50 μg /mL.

5. The reagent for measuring clotting time according to Claim 2, wherein the concentration of the non-human-derived component contained in the first reagent is 10 to 50 μg /mL.

6. The reagent for measuring clotting time according to Claim 1, comprising factor V and fibrinogen.

7. The reagent for measuring clotting time according to Claim 6, comprising first and second reagents, the first reagent containing the tissue factor, the factor V, the fibrinogen and the non-human-derived component, and the second reagent containing the calcium ion.

8. The reagent for measuring clotting time according to Claim 6 or 7, wherein the reagent for measuring clotting time is a combined factor-measuring reagent.

9. The reagent for measuring clotting time according to Claim 6, wherein the concentration of the non-human-derived component is 0.5 to 20 μg /mL.

10. The reagent for measuring clotting time according to Claim 7, wherein the concentration of the non-human-derived component in the first reagent is 0.5 to 20 μg /mL.

11. The reagent for measuring clotting time according to any Claim 1 - 10, wherein the vertebrates except human and pig.

12. The reagent for measuring clotting time according to Claim 1 - 11, wherein the tissue factor is selected from the group consisting of human-, rabbit-, bovine- and monkey-derived tissue factors.

13. The reagent for measuring clotting time according to Claim 12, wherein the tissue factor is a recombinant tissue factor.

14. A method for measuring clotting time, comprising the steps of;
    mixing a sample with a reagent comprising calcium ion, a tissue factor and a non-human-derived component selected from the group consisting of antibody, serum, plasma and immunoglobulin which derive from vertebrates except human, and
    measuring clotting time of the mixture.

15. The method of claim 14, wherein the reagent comprises a first reagent including the tissue factor and the non-human-derived component and a second reagent including the calcium ion, wherein the mixing step comprises the steps of first mixing the sample with the first reagent to obtain the first mixture and second mixing the first mixture with the second reagent to obtain the second mixture, and the measuring step comprises the step of measuring clotting time of the second mixture.

16. The method of claim 14, wherein the reagent further comprises factor V and fibrinogen.

**17.** The method of claim 16, wherein the reagent comprises a first reagent including the tissue factor, the factor V, the fibrinogen and the non-human-derived component and a second reagent including the calcium ion, wherein the mixing step comprises the steps of first mixing the sample with the first reagent to obtain the first mixture and second mixing the first mixture with the second reagent to obtain the second mixture, and the measuring step comprises the step of measuring clotting time of the second mixture.

**European Patent Office**

## EUROPEAN SEARCH REPORT

Application Number

EP 04 02 8118

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94/17415 A (DAHLBAECK BJOERN) 4 August 1994 (1994-08-04) * example 4 * ----- | 1-5,8-17 | G01N33/86 |
| X | BEVERS E M ET AL: "LUPUS ANTICOAGULANT IGG'S LA ARE NOT DIRECTED TO PHOSPHOLIPIDS ONLY BUT TO A COMPLEX OF LIPID-BOUND HUMAN PROTHROMBIN" THROMBOSIS AND HAEMOSTASIS, vol. 66, no. 6, 1991, pages 629-632, XP009042476 ISSN: 0340-6245 p. 630, col. 2, lines 43 -60 ----- | 1-17 | |
| X | SIMMELINK M J A ET AL: "A simple method to discriminate between beta2-glycoprotein I- and prothrombin-dependent lupus anticoagulants." JOURNAL OF THROMBOSIS AND HAEMOSTASIS : JTH. APR 2003, vol. 1, no. 4, April 2003 (2003-04), pages 740-747, XP009042760 ISSN: 1538-7933 p. 741/742, "Modified PTT-LA test" ----- | 14-17 | |
| A | WO 92/10586 A (LIPOSOME CO INC) 25 June 1992 (1992-06-25) abstract, claims 7 - 18 ----- | 1-17 | |

TECHNICAL FIELDS SEARCHED (Int.Cl.7)

G01N

The present search report has been drawn up for all claims

| Place of search | Date of completion of the search | Examiner |
|---|---|---|
| Munich | 18 January 2005 | Hoesel, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**   EP 04 02 8118

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2005

| Patent document cited in search report | | Publication date | Patent family member(s) | | | Publication date |
|---|---|---|---|---|---|---|
| WO 9417415 | A | 04-08-1994 | AT | 168781 | T | 15-08-1998 |
| | | | AU | 690535 | B2 | 30-04-1998 |
| | | | AU | 5982794 | A | 15-08-1994 |
| | | | BR | 9406223 | A | 09-01-1996 |
| | | | CA | 2154080 | A1 | 04-08-1994 |
| | | | CZ | 9501849 | A3 | 13-03-1996 |
| | | | DE | 69411898 | D1 | 27-08-1998 |
| | | | DE | 69411898 | T2 | 17-12-1998 |
| | | | DK | 690991 | T3 | 01-02-1999 |
| | | | EP | 0690991 | A1 | 10-01-1996 |
| | | | ES | 2119165 | T3 | 01-10-1998 |
| | | | FI | 953565 | A | 26-07-1995 |
| | | | GR | 3027697 | T3 | 30-11-1998 |
| | | | HU | 72191 | A2 | 28-03-1996 |
| | | | JP | 8506181 | T | 02-07-1996 |
| | | | NZ | 261190 | A | 19-12-1997 |
| | | | PL | 310050 | A1 | 13-11-1995 |
| | | | PL | 181638 | B1 | 31-08-2001 |
| | | | WO | 9417415 | A1 | 04-08-1994 |
| WO 9210586 | A | 25-06-1992 | AT | 140798 | T | 15-08-1996 |
| | | | AU | 648775 | B2 | 05-05-1994 |
| | | | AU | 7162091 | A | 08-07-1992 |
| | | | CA | 2095928 | A1 | 08-06-1992 |
| | | | DE | 69027936 | D1 | 29-08-1996 |
| | | | DE | 69027936 | T2 | 06-03-1997 |
| | | | DK | 561780 | T3 | 09-12-1996 |
| | | | EP | 0561780 | A1 | 29-09-1993 |
| | | | ES | 2090306 | T3 | 16-10-1996 |
| | | | GR | 3021412 | T3 | 31-01-1997 |
| | | | JP | 2983630 | B2 | 29-11-1999 |
| | | | JP | 6503640 | T | 21-04-1994 |
| | | | WO | 9210586 | A1 | 25-06-1992 |
| | | | US | 6261792 | B1 | 17-07-2001 |

For more details about this annex : see Official Journal of the European Patent Office, No. 12/82